# EUROPÄISCHE PATENTSCHRIFT

(11) **EP 1 487 573 B1**
(45) Veröffentlichungstag und Bekanntmachung des Hinweises auf die Patenterteilung: **09.09.2009**
(21) Anmeldenummer: 03743852.0
(22) Anmeldetag: 07.03.2003
(51) Int. Cl.: B01J 23/00, B01J 23/755, C07C 213/02, C07C 209/16, C07C 209/26, C07D 295/02, B01J 37/03, B01J 37/06

(54) **KATALYSATOREN UND VERFAHREN ZUR HERSTELLUNG VON AMINEN**
CATALYSTS AND METHOD FOR THE PRODUCTION OF AMINES
CATALYSEURS ET PROCEDE POUR PRODUIRE DES AMINES

(30) Priorität: 14.03.2002 DE 10211101
(43) Veröffentlichungstag der Anmeldung: 22.12.2004
(73) Patentinhaber: BASF SE, 67056 Ludwigshafen (DE)
(72) Erfinder: GERLACH, Till, 67071 Ludwigshafen (DE); FUNKE, Frank, 67067 Ludwigshafen (DE); MELDER, Johann-Peter, 67459 Böhl-Iggelheim (DE); ISELBORN, Stefan, 67069 Ludwigshafen (DE); RUDLOFF, Martin, 67273 Weisenheim (DE); HÜLLMANN, Michael, 64625 Bensheim (DE)
(86) Internationale Anmeldenummer: PCT/EP2003/002335
(87) Internationale Veröffentlichungsnummer: WO 2003/076386

(56) Entgegenhaltungen:
- EP-A- 0 017 651
- EP-A- 0 963 975

## Beschreibung

Die vorliegende Erfindung betrifft neue alkalimetallarme und alkalimetallfreie Katalysatoren enthaltend Zirkonium, Kupfer, Kobalt und Nickel sowie die Verwendung dieser Katalysatoren in Verfahren zur Herstellung von Aminen durch Umsetzung von primären oder sekundären Alkoholen, Aldehyden oder Ketonen bei erhöhter Temperatur und erhöhtem Druck mit Wasserstoff und Stickstoffverbindungen, ausgewählt aus der Gruppe Ammoniak, primäre und sekundäre Amine.

Aus der EP-A1-382 049 (BASF AG) sind Katalysatoren, die sauerstoffhaltige Zirkonium-, Kupfer-, Kobalt- und Nickelverbindungen enthalten, und Verfahren zur hydrierenden Aminierung von Alkoholen bekannt. Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele). Diese Katalysatoren zeichnen sich zwar durch eine gute Aktivität und Selektivität aus, besitzen allerdings verbesserungswürdige Standzeiten.

Aus der EP-A2-514 692 (BASF AG) sind Kupfer-, Nickel- und/oder Kobalt-, Zirkonium- und/oder Aluminiumoxid enthaltende Katalysatoren zur katalytischen Aminierung von Alkoholen in der Gasphase mit Ammoniak oder primären Aminen und Wasserstoff bekannt.

Diese Patentanmeldung lehrt, dass bei diesen Katalysatoren das Atomverhältnis von Nickel zu Kupfer 0,1 bis 1,0, bevorzugt 0,2 bis 0,5 (vergl. loc. cit.: Beispiel 1) betragen muss, da ansonsten bei der Aminierung von Alkoholen in erhöhtem Maße ausbeutemindernde Nebenprodukte auftreten (loc. cit.: Beispiele 6 und 12). Als Träger wird bevorzugt Aluminiumoxid (loc. cit.: Beispiele 1 bis 5 und 7 bis 11) verwendet.

Aus der EP-A1-696 572 und der EP-A-697 395 (beide BASF AG) sind Nickel-, Kupfer-, Zirkoniumoxid und Molybdänoxid enthaltende Katalysatoren zur katalytischen Aminierung von Alkoholen mit Stickstoffverbindungen und Wasserstoff bekannt. Mit diesen Katalysatoren werden zwar hohe Umsätze erzielt, aber es können sich Nebenprodukte (z.B. Ethylamin) bilden, die selbst bzw. deren Folgeprodukte in der Aufarbeitung stören.

EP-A2-905 122 (BASF AG) beschreibt ein Verfahren zur Herstellung von Aminen aus Alkoholen und Stickstoffverbindungen unter Verwendung eines Katalysators, dessen katalytisch aktive Masse sauerstoffhaltige Verbindungen des Zirkoniums, Kupfers und Nickels und keine sauerstoffhaltigen Verbindungen des Kobalts oder Molybdäns enthält.

EP-A-1 035 106 (BASF AG) betrifft die Verwendung von Katalysatoren enthaltend sauerstoffhaltige Verbindungen des Zirkoniums, Kupfers und Nickels zur Herstellung von Aminen durch aminierende Hydrierung von Aldehyden oder Ketonen.

EP-A1-963 975 und EP-A2-1 106 600 (beide BASF AG) beschreiben Verfahren zur Herstellung von Aminen aus Alkoholen bzw. Aldehyden oder Ketonen und Stickstoffverbindungen unter Verwendung eines Katalysators, dessen katalytisch aktive Masse 22-40 Gew.-% (bzw. 22-45 Gew.-%) sauerstoffhaltige Verbindungen des Zirkoniums, 1-30 Gew.-% sauerstoffhaltige Verbindungen des Kupfers und jeweils 15-50 Gew.-% (bzw. 5-50 Gew.-%) sauerstoffhaltige Verbindungen des Nickels und Kobalts enthält.

Beim Einsatz der sehr aktiven Katalysatoren der EP-A1-963 975 und EP-A2-1 106 600 kann es bei höheren Temperaturen zu verstärkten Decarbonylierungen der gegebenenfalls intermediär entstandenen Carbonylfunktion kommen. Die Bildung von Methan durch Hydrierung von Kohlenmonoxid (CO) führt aufgrund der großen freiwerdenden Hydrierwärme zu einer "Durchgehgefahr", d.h. einem unkontrolliertem Temperaturanstieg im Reaktor. Wird CO durch Amine abgefangen, kommt es zur Bildung von Methylnebenkomponenten. Bei der Aminierung von Diethylenglykol kommt es beispielsweise verstärkt zur Bildung von unerwünschtem Methoxyethanol bzw. Methoxyethylamin.

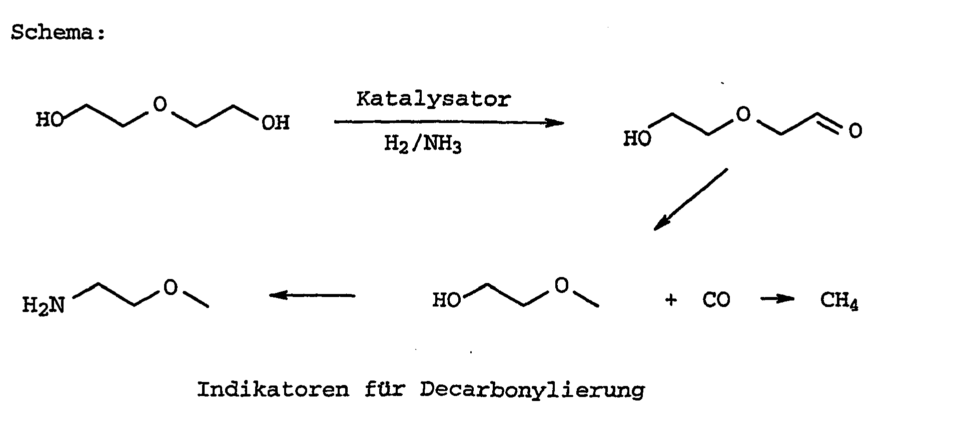

Als Reaktionsmechanismus der Aminierung von primären oder sekundären Alkoholen wird angenommen, dass der Alkohol zunächst an einem Metallzentrum zum entsprechenden Aldehyd dehydriert wird. Hierbei kommt dem Kupfer als Dehydrierkomponente vermutlich besondere Bedeutung zu. Werden Aldehyde zur Aminierung eingesetzt, entfällt dieser Schritt.

Der gebildete bzw. eingesetzte Aldehyd kann durch Reaktion mit Ammoniak oder primärem oder sekundärem Amin unter Wasserabspaltung und anschließender Hydrierung aminiert werden. Diese Kondensation des Aldehyds mit der o.g. Stickstoffverbindung wird vermutlich durch saure Zentren des Katalysators katalysiert. In einer unerwünschten Nebenreaktion kann der Aldehyd aber auch decarbonyliert werden, d.h. dass die Aldehydfunktion als CO abgespalten wird. Die Decarbonylierung bzw. Methanisierung findet vermutlich an einem metallischen Zentrum statt. Das CO wird an dem Hydrierkatalysator zu Methan hydriert, so dass die Methanbildung das Ausmaß der Decarbonylierung anzeigt. Durch die Decarbonylierung entstehen die oben erwähnten unerwünschten Nebenprodukte wie z.B. Methoxyethanol oder Methoxyethylamin.

Es handelt sich bei der erwünschten Kondensation des Aldehyds mit Ammoniak oder primärem oder sekundärem Amin und bei der unerwünschten Decarbonylierung des Aldehyds um Parallelreaktionen, von denen die erwünschte Kondensation säurekatalysiert ist, während die unerwünschte Decarbonylierung durch metallische Zentren katalysiert ist.

Der vorliegenden Erfindung lag die Aufgabe zugrunde, die Wirtschaftlichkeit bisheriger Verfahren zur hydrierenden Aminierung von Aldehyden oder Ketonen und der Aminierung von Alkoholen zu verbessern und den Nachteilen des Stands der Technik, insbesondere den o.g. Nachteilen, abzuhelfen. Es sollten Katalysatoren gefunden werden, die technisch in einfacher Weise herzustellen sind und die es erlauben, die o.g. Aminierungen mit hohem Umsatz, hoher Ausbeute, Selektivität, Katalysatorstandzeit bei gleichzeitig hoher mechanischer Stabilität des Katalysatorformkörpers und geringer 'Durchgehgefahr', durchzuführen. Die Katalysatoren sollen demnach eine hohe Aktivität und unter den Reaktionsbedingungen eine hohe chemische und mechanische Stabilität aufweisen.

Dementsprechend wurden Katalysatoren gefunden, deren katalytisch aktive Masse vor der Behandlung mit Wasserstoff 22 bis 40 Gew.-% sauerstoffhaltigen Verbindungen des Zirkoniums, berechnet als ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das Molverhältnis von Nickel zu Kupfer größer 1 ist, 15 bis 50 Gew.-% sauerstoffhaltige Verbindungen des Kobalts, berechnet als CoO, und weniger als 0,5 Gew.-% Alkalimetall (M), berechnet als Alkalimetalloxid (M₂O), enthält sowie deren vorteilhafte Verwendung zur Herstellung von Aminen durch Umsetzung von primären oder sekundären Alkoholen, Aldehyden oder Ketonen bei erhöhter Temperatur und erhöhtem Druck mit Wasserstoff und Stickstoffverbindungen, ausgewählt aus der Gruppe Ammoniak, primäre und sekundäre Amine.

Des weiteren wurde ein verbessertes Verfahren zur Herstellung von Aminen durch Umsetzung von primären oder sekundären Alkoholen, Aldehyden oder Ketonen bei erhöhter Temperatur und erhöhtem Druck mit Wasserstoff und Stickstoffverbindungen, ausgewählt aus der Gruppe Ammoniak, primäre und sekundäre Amine, in Gegenwart eines Katalysators, gefunden, welches dadurch gekennzeichnet ist, dass man die oben genannten erfindungsgemäßen Katalysatoren einsetzt. Erfindungsgemäß wurde erkannt, dass die Aktivität des Katalysators zur Aminierung von primären oder sekundären Alkoholen, Aldehyden oder Ketonen in Gegenwart von H₂, z.B. die Aminierung von Diethylenglykol mit Ammoniak zu Aminodiglykol und Morpholin, mit abnehmendem Alkalimetall-Gehalt, z.B. Natrium-Gehalt, der Zirkonium-Kupfer-Nickel-Kobalt-Katalysatoren zunimmt.

Gleichzeitig nimmt das Ausmaß der unerwünschten Decarbonylierungsreaktion ab.

Eine besonders geringe Tendenz zur unerwünschten Decarbonylierung wurde an Katalysatoren beobachtet, die weniger als 0,5 Gew.-%, insbesondere weniger als 0,35 Gew.-%, ganz besonders weniger als 0,2 Gew.-%, Alkalimetall, jeweils berechnet als Alkalimetalloxid, enthalten.

Der Alkalimetall-Gehalt kann z.B. durch die Dauer der Waschung des bei der Herstellung des Katalysators anfallenden Filterkuchens beeinflusst werden, wobei eine verlängerte Waschzeit zu einem verringerten Alkalimetall-Gehalt führt.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z. B. Stränge) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators vor der Behandlung mit Wasserstoff.

Die katalytisch aktive Masse des Katalysators ist als die Summe der Massen der katalytisch aktiven Bestandteile definiert und enthält, vor der Behandlung mit Wasserstoff, im wesentlichen die katalytisch aktiven Bestandteile sauerstoffhaltige Verbindungen des Zirkoniums, Kupfers, Nickels und Kobalts.

Die Summe der o.g. katalytisch aktiven Bestandteile, berechnet als ZrO₂, CuO, NiO und CoO, in der katalytisch aktive Masse vor der Behandlung mit Wasserstoff beträgt üblicherweise 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt >99 bis 100 Gew.-%.

Die sauerstoffhaltigen Verbindungen des Nickels, Kobalts und Kupfers, jeweils berechnet als NiO, CoO und CuO, sind im allgemeinen insgesamt in Mengen von 31 bis 78 Gew.-%, bevorzugt 44 bis 75 Gew.-%, besonders bevorzugt 55 bis 75 Gew.-%, in der katalytisch aktiven Masse (vor der Behandlung mit Wasserstoff) enthalten, wobei das Molverhältnis von Nickel zu Kupfer größer 1 ist.

Der Gehalt der katalytisch aktiven Masse der erfindungsgemäßen Katalysatoren vor der Behandlung mit Wasserstoff an Alkalimetall M, berechnet als Alkalimetalloxid M₂O, beträgt weniger als 0,5 Gew.-%, bevorzugt weniger als 0,35 Gew.-%, insbesondere weniger als 0,2 Gew.-%.

Bei den Alkalimetallen M handelt es sich um Li, Na, K, Rb und/ oder Cs, insbesondere um Na und/oder K, ganz besonders um Na.

Die erfindungsgemäßen Katalysatoren enthalten in ihrer katalytisch aktiven Masse vor der Behandlung mit Wasserstoff
22 bis 40 Gew.-%, bevorzugt 25 bis 40 Gew.-%, besonders bevorzugt 25 bis 35 Gew.-%, sauerstoffhaltige Verbindungen des Zirkoniums, berechnet als ZrO₂,
1 bis 30 Gew.-%, bevorzugt 2 bis 25 Gew.-%, besonders bevorzugt 5 bis 15 Gew.-%, sauerstoffhaltige Verbindungen des Kupfers, berechnet als CuO,
15 bis 50 Gew.-%, bevorzugt 21 bis 45 Gew.-%, besonders bevorzugt 25 bis 40 Gew.-%, sauerstoffhaltige Verbindungen des Nickels, berechnet als NiO, wobei das Molverhältnis von Nickel zu Kupfer größer 1, bevorzugt größer 1,2, besonders bevorzugt 1,8 bis 8,5, ist,
15 bis 50 Gew.-%, bevorzugt 21 bis 45 Gew.-%, besonders bevorzugt 25 bis 40 Gew.-%, sauerstoffhaltige Verbindungen des Kobalts, berechnet als CoO,
und weniger als 0,5 Gew.-%, bevorzugt weniger als 0,35 Gew.-%, insbesondere weniger als 0,2 Gew.-%, Alkalimetall M, berechnet als Alkalimetalloxid M₂O.

Zur Herstellung der Katalysatoren sind verschiedenerlei Verfahrensweisen möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel-, Kobalt- und Kupferkomponenten aus einer diese Elemente enthaltenden, wässrigen Salzlösung mittels Basen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und - silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Basen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den erfindungsgemäßen Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpresst und tempert (wärmebehandelt). Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Der Herstellung der erfindungsgemäßen alkalimetallarmen oder alkalimetallfreien Zirkonium-Kupfer-Nickel-Kobalt-Katalysatoren kann auch entsprechend nach den in der älteren DE-Anmeldung Nr. 10142635.6 vom 31.08.01 beschriebenen Verfahren, erfolgen.

Die hergestellten Katalysatoren können als solche gelagert werden. Vor ihrem Einsatz als Katalysatoren zur hydrierenden Aminierung von Alkoholen, Aldehyden oder Ketonen werden sie üblicherweise durch Behandlung mit Wasserstoff vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ein weiterer Vorteil der erfindungsgemäßen Katalysatoren ist deren mechanische Stabilität, d.h. deren Härte. Die mechanische Stabilität kann durch die Messung der sogenannten Seitendruckfestigkeit bestimmt werden. Hierzu wird der Katalysatorformkörper, z. B. die Katalysatortablette, zwischen zwei parallelen Platten mit zunehmender Kraft belastet, wobei diese Belastung z. B. auf der Mantelseite von Katalysatortabletten erfolgen kann, bis ein Bruch des Katalysatorformkörpers eintritt. Die beim Bruch des Katalysatorformkörpers registrierte Kraft ist die Seitendruckfestigkeit.

### Amine der Formel I

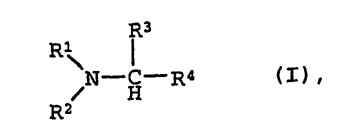

in der
- R¹, R²: Wasserstoff, C₁₋₂₀-Alkyl, C₃₋₁₂-Cycloalkyl, Aryl, C₇₋₂₀-Aralkyl und C₇₋₂₀-Alkylaryl oder gemeinsam (CH₂)ⱼ-X-(CH₂)ₖ,
- R³, R⁴: Wasserstoff, Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₁₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, R⁵-(OCR⁶R⁷CR⁸R⁹)ₙ-(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl und Y-(CH₂)ₘ-NR⁵-(CH₂)_{q} oder gemeinsam (CH₂)ₗ-X-(CH₂)ₘ oder
- R² und R⁴: gemeinsam (CH₂)ₗ-X-(CH₂)ₘ,
- R⁵, R¹⁰: Wasserstoff, C₁₋₄-Alkyl, C₇₋₄₀-Alkylphenyl,
- R⁶, R⁷, R⁸, R⁹: Wasserstoff, Methyl oder Ethyl,
- X: CH₂, CHR⁵, Sauerstoff (O), Schwefel (S) oder NR⁵,
- Y: N(R¹⁰)₂, Hydroxy, C₂₋₂₀-Alkylaminoalkyl oder C₃₋₂₀-Dialkylaminoalkyl,
- n: eine ganze Zahl von 1 bis 30 und
- j, k, l, m, q: eine ganze Zahl von 1 bis 4,
bedeuten, sind wirtschaftlich von besonderem Interesse.

Das erfindungsgemäße Verfahren findet daher bevorzugt zur Herstellung der Amine I Anwendung, indem man primäre oder sekundäre Alkohole der Formel II 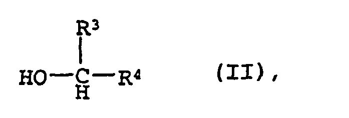 oder Aldehyde oder Ketone der Formel VI bzw. VII 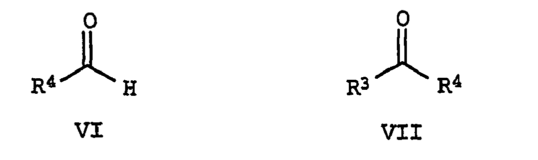 mit Stickstoffverbindungen der Formel III 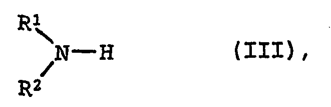 wobei R¹, R², R³ und R⁴ die oben genannten Bedeutungen haben, umsetzt.

Wie aus den Definitionen für die Reste R² und R⁴ hervorgeht, kann die Umsetzung auch intramolekular in einem entsprechenden Aminoalkohol, Aminoketon oder Aminoaldehyd erfolgen.

Zur Herstellung des Amins I wird demnach rein formal ein Wasserstoffatom des Amins III durch den Alkylrest R⁴(R³)CH- unter Freisetzung von einem Moläquivalent Wasser ersetzt.

Das erfindungsgemäße Verfahren findet auch bevorzugt Anwendung bei der Herstellung von zyklischen Aminen der Formel IV 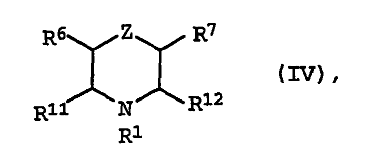 in der
- R¹¹ und R¹²: Wasserstoff, C₁- bis C₂₀-Alkyl, C₃- bis C₁₂-Cycloalkyl, Aryl, Heteroaryl, C₇- bis C₂₀-Aralkyl und C₇- bis C₂₀-Alkylaryl,
- Z: CH₂, CHR⁵, 0, NR⁵ oder NCH₂CH₂OH bedeuten und
- R¹, R⁶, R⁷: die oben genannten Bedeutungen haben,
durch Umsetzung von Alkoholen der Formel V 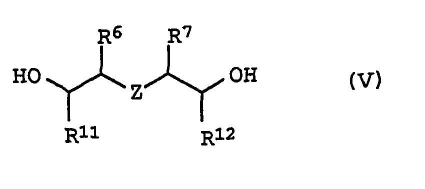 mit Ammoniak oder primären Aminen der Formel VI

R¹-NH₂ (VI).

Die Substituenten R¹ bis R¹², die Variablen X, Y, Z und die Indizes j, k, l, m, n und q in den Verbindungen I, II, III, IV, V und VI haben unabhängig voneinander folgende Bedeutungen:
R¹, R², R³, R⁴, R⁵, R⁶, R⁷, R⁸, R⁹, R¹⁰, R¹¹, R¹²:
   - Wasserstoff (H),
R³, R⁴:
   - C₁₋₂₀₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀₋₂₀₀-Alkyl, wie Polybutyl, Polyisobutyl, Polypropyl, Polyisopropyl und Polyethyl, besonders bevorzugt Polybutyl und Polyisobutyl,
   - C1₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
   - C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(Aminoethyl)aminoethyl und N-(Aminoethyl)aminomethyl,
   - C₂₋₂₀-Hydroxyalkylaminoalkyl, bevorzugt C₃₋₈-Hydroxyalkylaminoalkyl, wie (2-Hydroxyethylamino)methyl, 2-(2-Hydroxyethylamino)ethyl und 3-(2-Hydroxyethylamino)propyl,
   - C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, besonders bevorzugt C₂- bis C₄-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
   - C₃₋₃₀-Dialkylaminoalkyl, bevorzugt C₃₋₂₀-Dialkylaminoalkyl, besonders bevorzugt C₃₋₁₀-N,N-Dialkylaminoalkyl, wie N,N-Dimethylaminomethyl, 2-(N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-isopropylamino) ethyl, (R⁵)₂N-(CH₂)_{q},
   - C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, Methylaminoethyl, Ethylaminomethyl, Ethylaminoethyl und iso-Propylaminoethyl, (R⁵)HN-(CH₂)_{q},
   - Y-(CH₂)ₘ-NR⁵-(CH₂)_{q},
   - C₄₋₂₀-Heteroarylalkyl, wie Pyrid-2-yl-methyl, Furan-2-yl-methyl, Pyrrol-3-yl-methyl und Imidazol-2-yl-methyl,
   - C₄₋₂₀-Alkylheteroaryl, wie 2-Methyl-3-pyridinyl, 4,5-Dimethylimidazol-2-yl, 3-Methyl-2-furanyl und 5-Methyl-2-pyrazinyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹, R², R³, R⁴:
   - C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenylbutyl, 2-Phenyl-butyl, 3-Phenyl-butyl und 4-Phenyl-butyl, besonders bevorzugt Benzyl, 1-Phenethyl und 2-Phenethyl,
   - R³ und R⁴ oder R² und R⁴ gemeinsam eine -(CH₂)ₗ-X-(CH₂)ₘ- Gruppe, , wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,
R¹, R²:
   - C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁₋₄-Alkyl wie Methyl, Ethyl, n-Propyl, iso-Propyl, n-Butyl, iso-Butyl, sec-Butyl und tert.-Butyl,
   - R¹ und R² gemeinsam eine -(CH₂)ⱼ-X-(CH₂)ₖ- Gruppe, wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,
R⁵, R¹⁰:
   - C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
R6, R⁷, R⁸, R⁹:
   - Methyl oder Ethyl, bevorzugt Methyl,
R¹¹, R¹²:
   - C₁- bis C₂₀-Alkyl, C₃- bis C₁₂-Cycloalkyl, Aryl, Heteroaryl, C₇- bis C₂₀-Aralkyl und C₇- bis C₂₀-Alkylaryl, jeweils wie oben definiert,
X:
   - CH₂, CHR⁵, Sauerstoff (O), Schwefel (S) oder NR⁵, bevorzugt CH₂ und O,
Y:
   - N(R¹⁰)₂, bevorzugt NH₂ und N(CH₃)₂,
   - Hydroxy (OH),
   - C₂₋₂₀-Alkylaminoalkyl, bevorzugt C₂₋₁₆-Alkylaminoalkyl, wie Methylaminomethyl, Methylaminoethyl, Ethylaminomethyl, Ethylaminoethyl und iso-Propylaminoethyl,
   - C₃₋₂₀-Dialkylaminoalkyl, bevorzugt C₃₋₁₆-Dialkylaminoalkyl, wie Dimethylaminomethyl, Dimethylaminoethyl, Diethylaminoethyl, Di-n-propylaminoethyl und Di-iso-propylaminoethyl,
Z:
   - CH₂, CHR⁵, O, NR⁵ oder NCH₂CH₂OH,
j, l:
   - eine ganze Zahl von 1 bis 4 wie 1, 2, 3 und 4, bevorzugt 2 und 3, besonders bevorzugt 2,
k, m, q:
   - eine ganze Zahl von 1 bis 4 wie 1, 2, 3 und 4, bevorzugt 2, 3 und 4, besonders bevorzugt 2 und 3,
n:
   - eine ganze Zahl von 1 bis 10, bevorzugt eine ganze Zahl von 1 bis 8 wie 1, 2, 3, 4, 5, 6, 7 oder 8, besonders bevorzugt eine ganze Zahl von 1 bis 6.

Als Alkohole eignen sich praktisch alle primären und sekundären Alkohole mit aliphatischer OH-Funktion. Die Alkohole können geradkettig, verzweigt oder zyklisch sein. Sekundäre Alkohole werden ebenso aminiert wie primäre Alkohole. Hinsichtlich der Kohlenstoffzahl der aminierbaren Alkohole gibt es praktisch keine Beschränkungen. Die Alkohole können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Alkohole aminiert werden, so hat man es über die Steuerung der Reaktionsbedingungen in der Hand, Aminoalkohole, zyklische Amine oder mehrfach aminierte Produkte zu erhalten.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidinen).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethyleniminen).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidinen). Aus Diglykol kann demnach durch Aminierung mit NH₃ Monoaminodiglykol (= ADG = H₂N-CH₂CH₂-O-CH₂CH₂-OH), Diaminodiglykol oder besonders bevorzugt Morpholin erhalten werden. Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n-Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxyethylamino)propan-1-ol, 2-(N,N-Dimethylamino)ethanol, 2-(N,N-Diethylamino)ethanol, 2-(N,N-Di-n-propylamino)ethanol, 2-(N,N-Di-iso-propylamino)ethanol, 2-(N,N-Din-butylamino)ethanol, 2-(N,N-Di-iso-butylamino)ethanol, 2-(N,N-Di-sec.-butylamino)ethanol, 2-(N,N-Di-tert.-butylamino)ethanol, 3-(N,N-Dimethylamino)propanol, 3-(N,N-Diethylamino)propanol, 3-(N,N-Di-n-propylamino)propanol, 3-(N,N-Di-iso-propylamino)propanol, 3-(N,N-Di-n-butylamino)propanol, 3-(N,N-Di-iso-butylamino)propanol, 3-(N,N-Di-sec.-butylamino)propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polyisobutylalkohole, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, tert.-Butanol, Fettalkohole, Ethylenglykol, Diethylenglykol, 2-(2-Dimethylamino-ethoxy)ethanol, N-Methyldiethanolamin und 2-(2-Dimethylaminoethoxy)ethanol.

Als im erfindungsgemäßen Verfahren einsetzbare Ketone eignen sich praktisch alle aliphatischen und aromatischen Ketone. Die aliphatischen Ketone können geradkettig, verzweigt oder zyklisch sein, die Ketone können Heteroatome enthalten. Hinsichtlich der Kohlenstoffzahl der aminierbaren Ketone gibt es praktisch keine Beschränkungen. Die Ketone können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich praktisch alle aliphatischen und aromatischen Aldehyde. Die aliphatischen Aldehyde können geradkettig, verzweigt oder zyklisch sein, die Aldehyde können Heteroatome enthalten. Hinsichtlich der Kohlenstoffzahl der aminierbaren Aldehyde gibt es praktisch keine Beschränkungen. Die Aldehyde können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)acetaldehyd, 4-Formyltetrahydropyran, 3- Formyltetrahydrofuran, 5-Formylvaleronitril, Citronellal, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe bzw. die Aldehydgruppe bzw. die Ketogruppe zunächst in die primäre Aminogruppen (-NH₂) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z.B. Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Isopropylethylamin, n-Butylamin, Di-n-Butylamin, s-Butylamin,. Dis-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Das Aminierungsmittel kann bezüglich der zu aminierenden alkoholischen Hydroxylgruppe bzw. Aldehydgruppe bzw. Ketogruppe in stöchiometrischen, unter- oder überstöchiometrischen Mengen eingesetzt werden.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe, Aldehydgruppe oder Ketogruppe eingesetzt.

Höhere Überschüsse sowohl an Ammoniak als auch an primären oder sekundären Aminen sind möglich.

Das erfindungsgemäße Verfahren lässt sich diskontinuierlich oder bevorzugt kontinuierlich wie folgt durchführen, wobei der Katalysator bevorzugt als Festbett im Reaktor angeordnet ist.

Die Ausführungsform als Wirbelbettreaktion mit in auf- und abwirbelnder Bewegung befindlichem Katalysatormaterial ist jedoch ebenfalls möglich.

Die Aminierung der primären oder sekundären Alkoholgruppen, Aldehydgruppen oder Ketogruppen des Edukts kann in der Flüssigphase oder in der Gasphase durchgeführt werden. Bevorzugt ist das Festbettverfahren in der Gasphase.

Beim Arbeiten in der Flüssigphase leitet man die Edukte (Alkohol, Aldehyd oder Keton plus Ammoniak oder Amin) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 300°C, bevorzugt 120 bis 270°C, besonders bevorzugt 130 bis 250°C, insbesondere 170 bis 230°C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol, Aldehyd oder Keton pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol, Aldehyd oder Keton plus Ammoniak oder Amin) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung von Alkoholen betragen im allgemeinen 80 bis 300°C, bevorzugt 120 bis 270°C, besonders bevorzugt 160 bis 250°C. Die Reaktionstemperaturen bei der hydrierenden Aminierung von Aldehyden und Ketonen betragen im allgemeinen bei 80 bis 300°C, bevorzugt bei 100 bis 250°C. Es ist dabei sowohl eine Anströmung des Katalysatorfestbetts von oben als auch von unten möglich. Den erforderlichen Gasstrom erhält man bevorzugt durch eine Kreisgasfahrweise.

Die Katalysatorbelastung liegt im allgemeinen im Bereich von 0,01 bis 2, bevorzugt 0,05 bis 0,5, kg Alkohol, Aldehyd oder Keton pro Liter Katalysator (Schüttvolumen) und Stunde.

Der Wasserstoff wird der Reaktion im allgemeinen in einer Menge von 5 bis 400 1, bevorzugt in einer Menge von 50 bis 200 1 pro Mol Alkohol-, Aldehyd- oder Ketonkomponente zugeführt, wobei die Literangaben jeweils auf Normalbedingungen umgerechnet wurden (S.T.P.).

Die Aminierung von Aldehyden bzw. Ketonen unterscheidet sich in der Durchführung von der Aminierung von Alkoholen dadurch, dass bei der Aminierung von Aldehyden und Ketonen mindestens stöchiometrische Mengen an Wasserstoff vorhanden sein müssen.

Sowohl beim Arbeiten in der Flüssigphase als auch beim Arbeiten in der Gasphase ist die Anwendung höherer Temperaturen und höherer Gesamtdrücke möglich. Der Druck im Reaktionsgefäß, welcher sich aus der Summe der Partialdrücke des Aminierungsmittels, des Alkohols, Aldehyds bzw. Ketons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnten". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe, Aldehydgruppe bzw. Ketogruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte durch Destillation bzw. Rektifikation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Die erfindungsgemäß erhältlichen Amine eignen sich u. a. als Zwischenprodukte bei der Herstellung von Kraftstoffadditiven (US-A-3,275,554; DE-A-21 25 039 und DE-A-36 11 230), Tensiden, Arznei- und Pflanzenschutzmitteln sowie von Vulkanisationsbeschleunigern.

### Beispiele

### A) Herstellung von Zirkonium-Kupfer-Nickel-Kobalt-Katalysatoren mit Natriumgehalten von 0,11 bis 1,1 Gew.-%, berechnet als Natriumoxid

Zur Fällung wurde in einem konstanten Strom eine wässrige Lösung aus Nickelnitrat, Kupfernitrat, Kobaltnitrat und Zirkoniumacetat gleichzeitig mit einer 20 %igen wässrigen Natriumcarbonatlösung in ein Rührgefäß bei einer Temperatur von 70°C so zugegeben, dass der mit einer Glaselektrode gemessene pH-Wert in einem Bereich von 6,0 bis 7,0 aufrechterhalten wurde. Die Konzentration der Metallsalze in der Metallsalzlösung wurde so eingestellt, dass rechnerisch schließlich ein Katalysator mit einem Gewichtsverhältnis von NiO / CoO / CuO / ZrO₂ von 1 / 1 / 0,393 / 1,179 resultierte. Nach erfolgter Zugabe der Metallsalzlösung und der Natriumcarbonatlösung wurde noch eine Stunde bei 70°C nachgerührt und anschließend wurde der pH-Wert durch Zugabe von etwas Natriumcarbonatlösung auf einen Wert von 7,4 erhöht.

Die erhaltene Suspension wurde filtriert und der Filterkuchen mit vollentsalztem Wasser gewaschen. Durch unterschiedliche Waschzeiten, d.h. Verweilzeiten des Waschwassers am Filterkuchen, beziehungsweise durch unterschiedliche Mengen an Waschwasser resultierten Katalysatoren mit unterschiedlichen Natriumgehalten. Danach wurde der Filterkuchen bei einer Temperatur von 200°C in einem Trockenschrank oder einem Sprühtrockner getrocknet. Das auf diese Weise erhaltene Hydroxidcarbonatgemisch wurde nun bei einer Temperatur von 400°C über einen Zeitraum von 2 Stunden getempert.

Die so erhaltenen Katalysatorpulver A1 bis A5 hatten die Zusammensetzung:

| A1: |
|---|
| 27,97 Gew.-% Ni, berechnet als NiO, |
| 27,97 Gew.-% Co, berechnet als CoO, |
| 10,99 Gew.-% Cu, berechnet als CuO, |
| 32,96 Gew.-% Zr, berechnet als ZrO₂, |
| 0,11 Gew.-% Na, berechnet als Na₂O. |

| A2: |
|---|
| 27,97 Gew.-% Ni, berechnet als NiO, |
| 27,97 Gew.-% Co, berechnet als CoO, |
| 10,98 Gew.-% Cu, berechnet als CuO, |
| 32,96 Gew.-% Zr, berechnet als ZrO₂, |
| 0,12 Gew.-% Na, berechnet als Na₂O. |

| A3: |
|---|
| 27,96 Gew.-% Ni, berechnet als NiO, |
| 27,96 Gew.-% Co, berechnet als CoO, |
| 10,99 Gew.-% Cu, berechnet als CuO, |
| 32,95 Gew.-% Zr, berechnet als ZrO₂, |
| 0,14 Gew.-% Na, berechnet als Na₂O. |

| A4: |
|---|
| 27,91 Gew.-% Ni, berechnet als NiO, |
| 27,91 Ges.-% Co, berechnet als CoO, |
| 10,97 Gew.-% Cu, berechnet als CuO, |
| 32.,89 Gew.-% Zr, berechnet als ZrO₂, |
| 0,32 Gew.-% Na, berechnet als Na₂O. |

| A5 (nicht erfindungsgemäß): |
|---|
| 27,69 Gew.-% Ni, berechnet als NiO, |
| 27,69 Gew.-% Co, berechnet als CoO, |
| 10,88 Gew.-% Cu, berechnet als CuO, |
| 32,64 Gew.-% Zr, berechnet als ZrO₂, |
| 1,10 Gew.-% Na, berechnet als Na₂O. |

Der Alkalimetallgehalt wurde durch Atomspektrometrie bestimmt. Die untere analytische Nachweisgrenze für Alkalimetalle bei dieser Methode lag bei 0,01 Gew.-%.

Die Katalysatorpulver wurden jeweils mit 3 Gew.-% Graphit vermischt und zu 5 x 3 mm-Tabletten verformt.

Auf diese Art wurden fünf verschiedene Katalysatoren A1 bis A5 hergestellt, deren katalytisch aktive Masse Na-Gehalte von 0,11 Gew.-% bis 1,1 Gew.-%, jeweils berechnet als Natriumoxid (Na₂O), aufwiesen.

Nach erfolgter Tablettierung wurden die Tabletten jeweils für 2 h bei 400°C in einem Muffelofen nachcalciniert.

Vor Einbau des jeweiligen Katalysators in den Testreaktor wurde er reduziert und anschließend passiviert:

Zur Reduktion wurde der Katalysator in einem Wasserstoff/Stickstoffstrom auf Temperaturen zwischen 100 und 200°C aufgeheizt. Diese Temperatur wurde gehalten, bis alle Exothermien, die durch die exotherme Reduktion im Reduktionsofen entstehen und durch Thermoelemente entlang des Ofenrohres verfolgt wurden, abgeklungen waren. Anschließend wurde auf eine Endtemperatur von 280°C aufgeheizt und diese Temperatur für 6 h gehalten. Der Katalysator wurde unter einem Stickstoffstrom auf Raumtemperatur abgekühlt und dann mit einem verdünnten Sauerstoffstrom passiviert. Bei der Passivierung wurde darauf geachtet, dass die Temperatur im Reaktor an keiner Stelle über 50°C anstieg.

### B) Hydrierende Aminierungen unter Verwendung von Katalysatoren gemäß A)

### Beispiel 1

### Herstellung von Morpholin durch hydrierende Aminierung von Diglykol

### Allgemeine Arbeitsvorschrift:

In einen kontinuierlich betriebenen Hochdruckreaktor (Sumpffahrweise) wurden 100 cm³ Katalysator A eingebaut. Nach Verschließen des Reaktors wurden 20 Nl/h (N1 = Normliter = auf Normalbedingungen umgerechnetes Volumen) Wasserstoff über den Katalysator gefahren. Der Druck wurde auf 50 bar eingestellt. Anschließend wurde die Temperatur mit 2°C/Min. auf 180°C angehoben. Der Druck wurde danach auf 200 bar nachgeregelt. Schließlich wurden Diethylenglykol (60 g/h, 0,57 mol/h) und Ammoniak (60 g/h, 3,53 mol/h) zugefahren (Belastung: 0,6 kg Diethylenglykol / [l_{Katalysator} • h]). Die Reaktionstemperatur wurde zunächst 16 h auf 200°C gehalten. Dabei erfuhr der Katalysator die vollständige Aktivierung. Anschließend wurde die Reaktionstemperatur auf 180°C abgesenkt. Aus dem Reaktionsaustrag wurde nach dessen Entspannung überschüssiges Ammoniak abdestilliert.

Analytik : GC-Flächenprozentanalytik. Proben 1:10 mit Wasser verdünnt. 30 m RTX-5 Amine, 0,32 mm, 1,5 µm, Temperaturprogramm: 80°C/4 Min., dann mit 10°C/Min. auf 280°C, dann 280°C/5 Min.

Gemäß dieser allgemeinen Arbeitsvorschrift wurden die Katalysatoren mit unterschiedlichen Natriumgehalten A1 bis A5 eingesetzt.

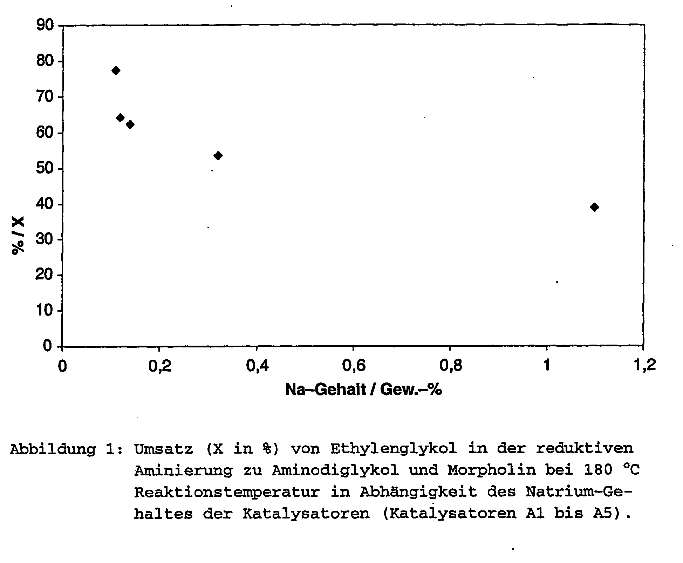

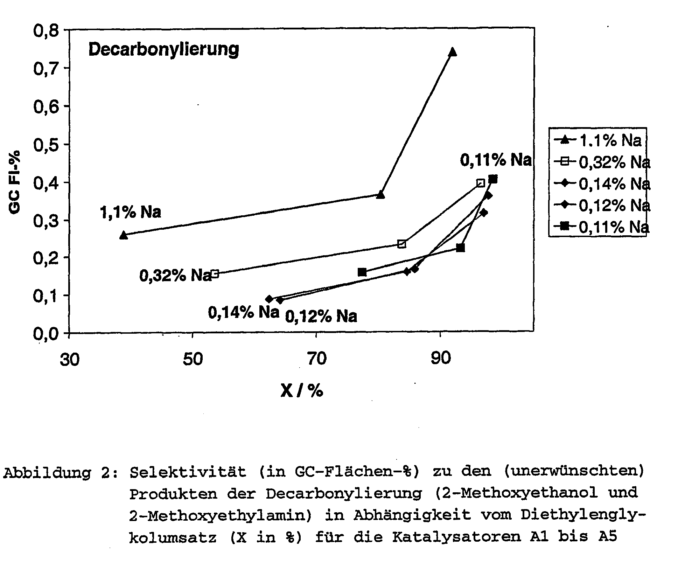

Die Ergebnisse sind in den folgenden Abbildungen 1 und 2 dargestellt.

Es ist zu erkennen, dass der bei einer Reaktionstemperatur von 180°C erzielte Umsatz und damit die Katalysatorproduktivität mit abnehmendem Na-Gehalt der Katalysatoren A5 bis A1 deutlich steigt (Abbildung 1). Die Katalysatoren sind also mit abnehmendem Na-Gehalt aktiver.

Weiterhin sinkt die - umsatzabhängige - Selektivität zur unerwünschten Decarbonylierung (Indikatoren: 2-Methoxyethanol und 2-Methoxyethylamin) mit abnehmendem Na-Gehalt (Abbildung 2).

### Beispiel 2

### Herstellung von Morpholin durch hydrierende Aminierung von Diglykol

Nach der allgemeinen Versuchsvorschrift gemäß Beispiel 1 wurden die zwei Katalysatoren A2 und A5 bei gleichem Diglykolumsatz verglichen. Dazu wurde Katalysator A2 (Na-Gehalt: 0,12 %) bei einer Reaktionstemperatur von 190°C gefahren. Um den gleichen Umsatz (bezogen auf Diethylenglykol) zu erreichen, musste der Katalysator A5 (Na-Gehalt: 1,10 %) bei einer Reaktionstemperatur von 200°C gefahren werden.

Die Ergebnisse sind in der folgenden Tabelle zusammengestellt.

Katalysator A2 zeigte eine höhere Gesamtselektivität zu den beiden Wunschprodukten (Morpholin und Aminodiglykol). Die Bildung von Methoxyethanol und Methoxyethylamin, jeweils Indikatoren für unerwünschte Nebenreaktionen, war bei Katalysator A2 um den Faktor 4 geringer als bei Katalysator A5.

| Katalysator | T °C | Umsatz % | S (MOR) % | S (ADG) % | S (MOR+ ADG) % | EtNH2 | MeOEtOH | MeOEtNH2 | Na-Gehalt Gew.-% |
|---|---|---|---|---|---|---|---|---|---|
| A2 | 190 | 93,2 | 77,7 | 9,89 | 87,6 | 0,1 | 0,07 | 0,08 | 0,12 |
| A5 | 200 | 92,9 | 80,15 | 6,64 | 86,8 | 0,14 | 0,3 | 0,28 | 1,10 |

| | | | | | | | | | |
|---|---|---|---|---|---|---|---|---|---|
| S = Selektivität (bezogen auf umgesetztes Diglykol MOR = Morpholin ADG = Aminodiglykol (H₂N(CH₂)₂O(CH₂)₂OH) MeOEtOH = 2-Methoxyethanol MeOEtNH2 = 2-Methoxyethylamin | | | | | | | | | |

### Beispiel 3

Aminierung von hydroformyliertem Polyisobuten

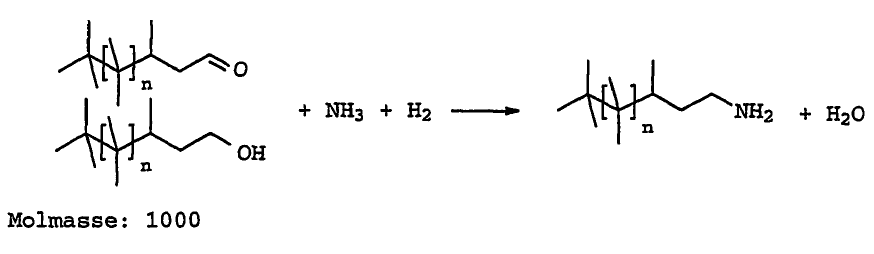

Die Versuche wurden in einem 1 m³-Rohrreaktor an einem Katalysator mit der Zusammensetzung

| |
|---|
| 28,0 Gew.-% Ni, berechnet als NiO, |
| 28,0 Gew.-% Co, berechnet als CoO, |
| 11,0 Gew.-% Cu, berechnet als CuO, |
| 32,99 Gew.-% Zr, berechnet als ZrO₂, |
| 0,01 Gew.-% Na, berechnet als Na₂O, |

der analog der obigen Vorschrift in Beispiel A hergestellt wurde, durchgeführt (Sumpffahrweise). Der Katalysator wurde reduziert/ passiviert eingebaut.

Zunächst wurde der Reaktor 3 x mit 40 bar N₂ gespült. Nach einer Dichtheitsprüfung bei 200 bar N₂, wurde auf 120 bar entspannt. Der Kreisgaskompressor wurde mit 1000 Nm3/h N₂ in Betrieb genommen und bei Umgebungstemperatur wurde mit der Ammoniakbenetzung begonnen. Bei einer Temperatur von ca. 120°C wurde der Ammoniakzulauf kurz abgestellt, wobei ein weiterer Temperaturanstieg bis auf maximal 183°C beobachtet wurde. Nach Abkühlung wurde die Ammoniakmenge stufenweise auf den Zielwert für die Synthese von 250 kg/h eingestellt und auf 190°C aufgeheizt. Während der Ammoniakfahrt wurde mit H₂-Zudosierung angefangen und auf 200 bar aufgepresst. Hierbei wurden folgende Parameter eingestellt.

| Druck (bar) | Temp. (°C) | Kat.Bel. (kg/(l•h)) | Ammoniak (kg/h) | Kreisgasmenge (Nm³/h) |
|---|---|---|---|---|
| 200 | 200 | 0,5 - 1,2 | 1500 | 1500 |
| 200 | 180 | 0,5 - 0,9 | 250 | 300 |

Die Ergebnisse sind in der folgenden Tabelle aufgeführt.

| Laufzeit (h) | T (°C) | Zulauf (kg/h) | Ammoniak (kg/h) | Kreis-gas (Nm³/h) | AZ (mg KOH/g) | AC (mg KOH/g) | s+t AZ (mg KOH/g) |
|---|---|---|---|---|---|---|---|
| 21 | 176 | 250 | 250 | 1200 | 16,4 | 17,0 | 5,8 |
| 45 | 180 | 500 | 250 | 300 | 20,6 | 20,6 | 2,1 |
| 69 | 180 | 600 | 250 | 300 | 21,0 | 21,0 | 1,8 |
| 93 | 180 | 700 | 250 | 300 | 21,0 | 21,5 | 1,6 |
| 141 | 180 | 800 | 250 | 300 | 21,5 | 21,8 | 1,5 |
| 165 | 180 | 900 | 250 | 300 | 21,4 | 21,9 | 1,1 |
| 189 | 177 | 900 | 250 | 300 | 21,6 | 22,1 | 1,1 |
| 213 | 176 | 900 | 250 | 300 | 21,4 | 22,0 | 0,9 |
| 237 | 178 | 800 | 250 | 300 | 21,5 | 21,8 | 1,2 |
| 333 | 176 | 800 | 250 | 300 | 21,6 | 22,5 | 1,0 |
| 357 | 173 | 800 | 250 | 300 | 21,5 | 22,4 | 0,9 |
| 381 | 172 | 700 | 250 | 300 | 21,4 | 22,0 | 1,0 |
| 405 | 170 | 700 | 250 | 300 | 21,1 | 21,9 | 0,8 |
| 453 | 171 | 700 | 250 | 300 | 21,5 | 22,2 | 0,9 |
| 477 | 169 | 600 | 250 | 300 | 21,5 | 22,0 | 0,9 |
| 525 | 168 | 300 | 250 | 300 | 20,9 | 21,2 | 1,6 |

### Erklärung der Abkürzungen:

Kat. Bel. = Katalysatorbelastung in kg (Alkohol + Aldehyd) pro Liter Katalysator und pro Stunde.

Nm³ = Normkubikmeter = auf Normalbedingungen umgerechnetes Volumen.

s+t AZ steht für Aminzahl bezüglich alkylierter sekundärer und tertiärer Amine.

(Die AZ wird nach bekannter Methode durch eine Säure-Base-Titration ermittelt. Explizit wird die Base mit HCl titriert). Die AZ gilt als Maß des Aminierungsgrades.

AC steht für Acetylierungszahl.

(Zur Ermittlung der AC wird nach bekannter Methode die Probe mit einem Acetylierungsgemisch, bestehend aus Essigsäureanhydrid (ESA), Eisessig und Pyridin, bei Raumtemperatur umgesetzt.

In den vorliegenden Fällen reagiert die Base mit ESA zum Amid. Der Rest-Überschuss ESA wird mit H₂O zur Essigsäure umgesetzt, die wiederum mit NaOH zurücktitriert).

Mit der AC bestimmt man das Gesamtpotential an funktionellen Gruppen des Produktes. Mit der AZ den entsprechenden Aminanteil daraus.

## Patentansprüche

1. Verfahren zur Herstellung von Aminen durch Umsetzung von primären oder sekundären Alkoholen, Aldehyden oder Ketonen bei erhöhter Temperatur und erhöhtem Druck mit Wasserstoff und Stickstoffverbindungen, ausgewählt aus der Gruppe Ammoniak, primäre und sekundäre Amine, in Gegenwart eines Katalysators, **dadurch gekennzeichnet, dass** die katalytisch aktive Masse des Katalysators vor der Behandlung mit Wasserstoff
22 bis 40 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das Molverhältnis von Nickel zu Kupfer größer 1 ist,
15 bis 50 Gew.-% sauerstoffhaltige Verbindungen des Kobalts, berechnet als CoO,
und weniger als 0,5 Gew.-% Alkalimetall, berechnet als Alkalimetalloxid, enthält.

2. Verfahren nach Anspruch 1, **dadurch gekennzeichnet, dass** die katalytisch aktive Masse des Katalysators vor der Behandlung mit Wasserstoff weniger als 0,35 Gew.-% Alkalimetall, berechnet als Alkalimetalloxid, enthält.

3. Verfahren nach Anspruch 1, **dadurch gekennzeichnet, dass** die katalytisch aktive Masse des Katalysators vor der Behandlung mit Wasserstoff weniger als 0,2 Gew.-% Alkalimetall, berechnet als Alkalimetalloxid, enthält.

4. Verfahren nach einem der Ansprüche 1 bis 3, **dadurch gekennzeichnet, dass** die katalytisch aktive Masse des Katalysators vor der Behandlung mit Wasserstoff
25 bis 40 Gew.-% sauerstoffhaltige Verbindungen des Zirkoniums, berechnet als ZrO₂, Zeichn.
2 bis 25 Gew.-% sauerstoffhaltige Verbindungen des Kupfers, berechnet als CuO,
21 bis 45 Gew.-% sauerstoffhaltige Verbindungen des Nickels, berechnet als NiO, wobei das Molverhältnis von Nickel zu Kupfer größer 1 ist, und
21 bis 45 Gew.-% sauerstoffhaltige Verbindungen des Kobalts, berechnet als CoO, enthält.

5. Verfahren nach einem der Ansprüche 1 bis 4, **dadurch gekennzeichnet, dass** man die Umsetzung bei Temperaturen von 80 bis 300°C durchführt.

6. Verfahren nach einem der Ansprüche 1 bis 5, **dadurch gekennzeichnet, dass** man die Umsetzung in der Flüssigphase bei Drücken von 5 bis 30 MPa oder in der Gasphase bei Drücken von 0,1 bis 40 MPa durchführt.

7. Verfahren nach einem der Ansprüche 1 bis 6 zur Herstellung Aminen der Formel I 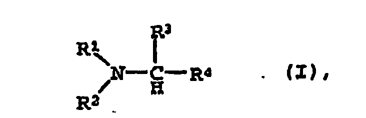 in der
R¹, R² Wasserstoff, C₁₋₂₀-Alkyl, C₃₋₁₂-Cycloalkyl, Aryl, C₇₋₂₀-Aralkyl und C₇₋₂₀-Alkylaryl oder gemeinsam (CH₂)ⱼ-X-(CH₂)ₖ.
R³, R⁴ Wasserstoff, Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₁₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl,
R⁵-(OCR⁶R⁷CR⁸R⁹)ₙ-(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl und Y-(CH₂)ₘ-NR⁵-(CH₂)_{q} oder gemeinsam (CH₂)ₗ-X-(CH₂)ₘ oder
R² und R⁴ gemeinsam (CH₂)ₗ-X-(CH₂)ₘ,
R⁵, R¹⁰ Wasserstoff, C₁₋₄-Alkyl, C₇₋₄₀-Alkylphenyl,
R⁶, R⁷, R⁸, R⁹ Wasserstoff, Methyl oder Ethyl,
X CH₂, CHR⁵, Sauerstoff (O), Schwefel (S) oder NR⁵,
Y N(R¹⁰)₂, Hydroxy, C₂₋₂₀-Alkylaminoalkyl oder C₃₋₂₀-Dialkylaminoalkyl,
n eine ganze Zahl von 1 bis 30 und
j, k, l, m, q eine ganze Zahl von 1 bis 4
bedeuten, durch Umsetzung von primären oder sekundären Alkoholen der Formel II 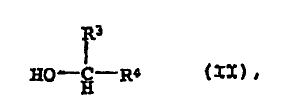 oder Aldehyden oder Ketonen der Formel VI bzw. VII 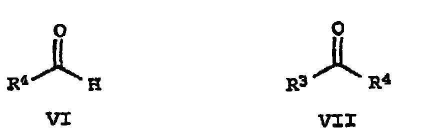 mit Stickstoffverbindungen der Formel III

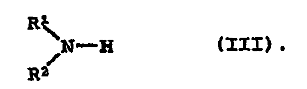

8. Katalysatoren wie in einem der Ansprüche 1 bis 4 definiert.

## Claims

1. A process for preparing amines by reacting primary or secondary alcohols, aldehydes or ketones with hydrogen and nitrogen compounds selected from the group consisting of ammonia and primary and secondary amines at elevated temperature and superatmospheric pressure in the presence of a catalyst whose catalytically active composition prior to treatment with hydrogen comprises
from 22 to 40% by weight of oxygen-containing compounds of zirconium, calculated as ZrO₂,
from 1 to 30% by weight of oxygen-containing compounds of copper, calculated as CuO,
from 15 to 50% by weight of oxygen-containing compounds of nickel, calculated as NiO, with the molar ratio of nickel to copper being greater than 1,
from 15 to 50% by weight of oxygen-containing compounds of cobalt, calculated as CoO,
and less than 0.5% by weight of alkali metal, calculated as alkali metal oxide.

2. The process according to claim 1, wherein the catalytically active composition of the catalyst prior to treatment with hydrogen comprises less than 0.35% by weight of alkali metal, calculated as alkali metal oxide.

3. The process according to claim 1, wherein the catalytically active composition of the catalyst prior to treatment with hydrogen comprises less than 0.2% by weight of alkali metal, calculated as alkali metal oxide.

4. The process according to any of claims 1 to 3, wherein the catalytically active composition of the catalyst prior to treatment with hydrogen comprises
from 25 to 40% by weight of oxygen-containing compounds of zirconium, calculated as ZrO₂,
from 2 to 25% by weight of oxygen-containing compounds of copper, calculated as CuO,
from 21 to 45% by weight of oxygen-containing compounds of nickel, calculated as NiO, with the molar ratio of nickel to copper being greater than 1, and
from 21 to 45% by weight of oxygen-containing compounds of cobalt, calculated as CoO.

5. The process according to any of claims 1 to 4, wherein the reaction is carried out at from 80 to 300°C.

6. The process according to any of claims 1 to 5, wherein the reaction is carried out in the liquid phase at pressures of from 5 to 30 MPa or in the gas phase at pressures of from 0.1 to 40 MPa.

7. The process according to any of claims 1 to 6 for preparing amines of the formula I 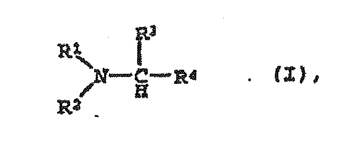 where
R¹, R² are each hydrogen, C₁₋₂₀-alkyl, C₃₋₁₂-cycloalkyl, aryl, C₇₋₂₀-aralkyl and C₇₋₂₀-alkylaryl or together represent (CH₂)ⱼ-X-(CH₂)ₖ,
R³, R⁴ are each hydrogen, alkyl such as C₁₋₂₀₀-alkyl, cycloalkyl such as C₃₋₁₂-cycloalkyl, hydroxyalkyl such as C₁₋₂₀-hydroxyalkyl, aminoalkyl such as C₁₋₂₀-aminoalkyl, hydroxyalkylaminoalkyl such as C₁₋₂₀- hydroxyalkylaminoalkyl, alkoxyalkyl such as C₂₋₃₀-alkoxyalkyl, dialkylaminoalkyl such as C₃₋₃₀-dialkylaminoalkyl, alkylaminoalkyl such as C₂₋₃₀-alkylaminoalkyl, R⁵-(OCR⁶R⁷CR⁸R⁹)ₙ-(OCR⁶R⁷), aryl, heteroaryl, aralkyl such as C₇₋₂₀-aralkyl, heteroarylalkyl such as C₄₋₂₀-heteroarylalkyl, alkylaryl such as C₇₋₂₀-alkylaryl, alkylheteroaryl such as C₄₋₂₀-alkylheteroaryl and Y-(CH₂)ₘ-NR⁵-(CH₂)_{q} or together represent (CH₂)₁-X-(CH₂)ₘ or
R² and R⁴ together represent (CH₂)₁-X-(CH₂)ₘ,
R⁵, R¹⁰ are each hydrogen, C₁₋₄-alkyl, C₇₋₄₀-alkylphenyl,
R⁶, R⁷, R⁸, R⁹ are each hydrogen, methyl or ethyl,
X is CH₂, CHR⁵, oxygen (O), sulfur (S) or NR⁵,
Y is N(R¹⁰)₂, hydroxy, C₂₋₂₀-alkylaminoalkyl or C₃₋₂₀-dialkylaminoalkyl,
n is an integer from 1 to 30 and
j, k, l, m, q are each an integer from 1 to 4,
by reacting primary or secondary alcohols of the formula II 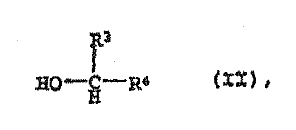 or aldehydes or ketones of the formula VI or VII 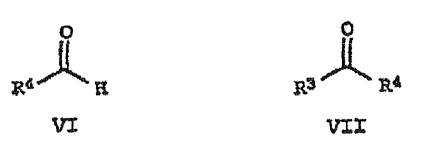 with nitrogen compounds of the formula III

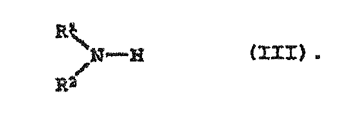

8. A catalyst as defined in any of claims 1 to 4.

## Revendications

1. Procédé pour la préparation d'amines par transformation d'alcools primaires ou secondaires, d'aldéhydes ou de cétones à température élevée et pression élevée avec de l'hydrogène et des composés azotés, choisis dans le groupe formé par l'ammoniaque, les amines primaires et secondaires, en présence d'un catalyseur, **caractérisé en ce que** la masse catalytiquement active du catalyseur contient, avant le traitement avec l'hydrogène
- 22 à 40 % en poids de composés oxygénés du zirconium, calculés en ZrO₂,
- 1 à 30 % en poids de composés oxygénés du cuivre, calculés en CuO,
- 15 à 50% en poids de composés oxygénés du nickel, calculés en NiO, le rapport molaire de nickel à cuivre étant supérieur à 1,
- 15 à 50 % en poids de composés oxygénés du cobalt, calculés en CoO,
- et moins de 0,5% en poids de métal alcalin, calculé en oxyde de métal alcalin.

2. Procédé selon la revendication 1, **caractérisé en ce que** la masse catalytiquement active du catalyseur contient, avant le traitement avec l'hydrogène, moins de 0,35% en poids de métal alcalin, calculé en oxyde de métal alcalin.

3. Procédé selon la revendication 1, **caractérisé en ce que** la masse catalytiquement active du catalyseur contient, avant le traitement avec l'hydrogène, moins de 0,2% en poids de métal alcalin, calculé en oxyde de métal alcalin.

4. Procédé selon l'une quelconque des revendications 1 à 3, **caractérisé en ce que** la masse catalytiquement active du catalyseur contient, avant le traitement avec l'hydrogène
- 25 à 40 % en poids de composés oxygénés du zirconium, calculés en ZrO₂,
- 2 à 25 % en poids de composés oxygénés du cuivre, calculés en CuO,
- 21 à 45% en poids de composés oxygénés du nickel, calculés en NiO, le rapport molaire de nickel à cuivre étant supérieur à 1, et
- 21 à 45 % en poids de composés oxygénés du cobalt, calculés en CoO.

5. Procédé selon l'une quelconque des revendications 1 à 4, **caractérisé en ce qu'**on réalise la transformation à des températures de 80 à 300°C.

6. Procédé selon l'une quelconque des revendications 1 à 5, **caractérisé en ce qu'**on réalise la transformation en phase liquide à des pressions de 5 à 30 MPa ou en phase gazeuse à des pressions de 0,1 à 40 MPa.

7. Procédé selon l'une quelconque des revendications 1 à 6 pour la préparation d'amines de formule I 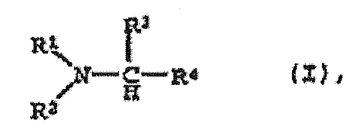 dans laquelle
R¹, R² signifient hydrogène, C₁₋₂₀-alkyle, C₃₋₁₂-cycloalkyle, aryle, C₇₋₂₀-aralkyle et C₇₋₂₀-alkylaryle ou ensemble (CH₂)ⱼ-X-(CH₂)ₖ,
R³, R⁴ signifient hydrogène, alkyle, tel que C₁₋₂₀₀-alkyle, cycloalkyle, tel que C₃₋₁₂-cycloalkyle, hydroxyalkyle, tel que C₁₋₂₀-hydroxyalkyle, aminoalkyle, tel que C₁₋₂₀-aminoalkyle, hydroxyalkylaminoalkyle, tel que C₁₋₂₀-hydroxyalkylaminoalkyle, alcoxyalkyle, tel que C₂₋₃₀-alcoxyalkyle, dialkylaminoalkyle, tel que C₃₋₃₀-dialkylaminoalkyle, alkylaminoalkyle, tel que C₂₋₃₀-alkylaminoalkyle, R⁵-(OCR⁶R⁷CR⁸R⁹)ₙ-(OCR⁶R⁷), aryle, hétéroaryle, aralkyle, tel que C₇₋₂₀-aralkyle, hétéroarylalkyle, tel que C₄₋₂₀-hétéroarylalkyle, alkylaryle, tel que C₇₋₂₀-alkylaryle, alkylhétéroaryle, tel que C₄₋₂₀-alkylhétéroaryle et Y-(CH₂)ₘ-NR⁵-(CH₂)_{q} ou ensemble (CH₂)₁-X-(CH₂)ₘ ou
R² et R⁴ signifient ensemble (CH₂)ₗ-X-(CH₂)ₘ,
R⁵, R¹⁰ signifient hydrogène, C₁₋₄-alkyle, C₇₋₄₀-alkylphényle,
R⁶, R⁷, R⁸, R⁹ signifient hydrogène, méthyle ou éthyle,
X signifie CH₂, CHR⁵, oxygène (O), soufre (S) ou NR⁵,
Y signifie N(R¹⁰)₂, hydroxy, C₂₋₂₀-alkylaminoalkyle ou C₃₋₂₀-dialkylaminoalkyle,
n vaut un nombre entier de 1 à 30 et
j, k, l, m, q valent un nombre entier de 1 à 4,
par transformation d'alcools primaires et secondaires de formule II 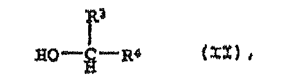 ou d'aldéhydes ou de cétones de formule VI ou VII 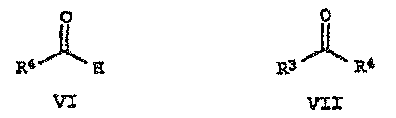 avec des composés azotés de formule III

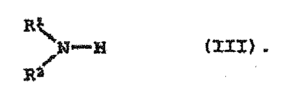

8. Catalyseurs tels que définis dans l'une quelconque des revendications 1 à 4.
